# EUROPEAN PATENT SPECIFICATION

(11) **EP 1 174 134 B1**
(45) Date of publication and mention of the grant of the patent: **15.06.2005**
(21) Application number: 00921064.2
(22) Date of filing: 27.04.2000
(51) Int. Cl.: A61K 31/4045, A61P 9/10, A61P 25/02, A23L 1/30

(54) **MELATONIN FOR TREATING PARALYSIS CAUSED BY CEREBRAL INFARCTION**
MELATONIN ZUR BEHANDLUNG VON PARALYSEN AUSGELÖST DURCH HIRNSCHLAG
MELATONINE POUR LE TRAITEMENT DES PARALYSIES CAUSEES PAR L'INFARCTUS CEREBRAL

(30) Priority: 28.04.1999 US 300456
(43) Date of publication of application: 23.01.2002
(73) Proprietor: Ajinomoto Co., Inc., Tokyo 104-8315 (JP)
(72) Inventor: NISHINO, Hitoo, Nagoya-shi, Aichi 468-0021 (JP); BORLONGAN, Cesario, V., Silver Spring, MD 20910 (US); UNEYAMA, Hisayuki, Ajinomoto Co., Inc., Kawaski-shi, Kanagawa 210-8681 (JP)
(74) Representative: Nicholls, Kathryn Margaret
(86) International application number: PCT/JP2000/002779
(87) International publication number: WO 2000/066118

(56) References cited:
- WO-A-98/21947
- WO-A-98/36735
- WO-A1-97/20555
- WO-A1-98/09653
- WO-A1-98/42667
- GUERRERO J M ET AL: "MELATONIN PREVENTS INCREASES IN NEURAL NITRIC OXIDE AND CYCLIC GMP PRODUCTION AFTER TRANSIENT BRAIN ISCHEMIA AND REPERFUSION IN THE MONGOLIAN GERBIL (MERIONES UNGUICULATUS)" JOURNAL OF PINEAL RESEARCH, MUNKSGAARD, COPENHAGEN, DK, vol. 23, no. 1, August 1997 (1997-08), pages 24-31, XP008008632 ISSN: 0742-3098
- SEIF-EL-NASR M ET AL: "Possible neuroprotective effects of melatonin against ischaemia/reperfusion insult in rat brain." MEDICAL SCIENCE RESEARCH, vol. 27, no. 9, 1999, pages 605-608, XP009016640 ISSN: 0269-8951
- LI XUE-JUN ZHANG LING-MEI ET AL: "Melatonin decreases production of hydroxyl radical during cerebral ischemia-reperfusion." ACTA PHARMACOLOGICA SINICA, vol. 18, no. 5, 1997, pages 394-396, XP009016636 ISSN: 0253-9756
- LING XIANG ET AL.: 'Protective effect of melatonin on injuried cerebral neurons is associated with bcl-2 protein over-expression' ACTA PHARMACOL. SIN. vol. 20, no. 5, 1999, pages 409 - 414, XP002929696

## Description

### Field of the Invention

The present invention relates to the use of melatonin for the production of a composition for the treatment of paralysis of the extremities caused by cerebral infarction.

### Background and Prior Art

Cerebral infarction is a severe cerebral dysfunction induced by cerebral ischemia or hypoxemia. With the increase of aged population, the incidence of diseases or conditions, which may cause cerebral ischemia such as cerebral thrombosis and cerebral embolism, is increasing. Indeed, the number of patients suffering from cerebral infarction and having cerebral dysfunctions is continuously increasing.

One of the cerebral dysfunctions caused by cerebral infarction is paralysis of the extremities. When the hands and legs cannot perform their functions, the quality of life of such affected patients is significantly lowered and many burdens are placed on them and their families. For such patients, complete daily care is necessary.

For the treatment of cerebral infarction, the use of a neuroprotective (e.g., as a glutamic acid antogonist, a channel antagonist, a radical scavenger, a neurogrowth factor, and a GABA agonist) has been proposed, but it requires further basic study. A central muscle relaxant, an agent for improving microcirculations (e.g., an anti-platelet drug and an agent for accelerating deformation of erythrocytes), an agent for improving central metabolism (e.g., an agent for enhancing the GABA system or the dopamine system and an agent for regulating the acetylcholine system), and the like have been complementarily administered in order to reduce adverse influences of cerebral infarction. Further, physiotherapies, such as a motorpathy, have been conducted in order to treat motor dysfunctions of extremities caused by cerebral infarction.

Melatonin (N-[2-(5-methoxy-1H-indol-3-yl)ethyl]acetamide) is secreted from the pineal gland, and acts upon the suprachiasmatic nucleus to influence the formation of a diurnal rhythm (e.g., Chem. & Eng. News 45: 40 (1967)).

Since melatonin is characterized by the above physiological action, it is used for the treatment of disorders of the diurnal rhythm, such as sleep disorders, emotional disorders, immune hypofunctions, caused by, for example, time-difference (jetlag) (e.g., Barchas et al., Nature 214: 919 (1967) and A. Miles, D. Philbrick, CRC Crit. Rev. Clin. Lab. Sci. 25: 231-253 (1987)).

It is also reported that melatonin has antioxidative activity and functions as a free radical scavenger *in vivo* (Life Sci. 60(25), 2255-2271 (1997)).

The possibility that administered melatonin inhibits brain nitric oxide (NO) production after transient cerebral ischemia/reperfusion and reduces brain damage caused by free radicals is mentioned (Guerrero J M. et al., J. Pineal Res. 23(1), 24-31(1997)).

Further, Sunghee Cho et al. (Brain Res. 755(2), 335-338(1997)) describes that intraperitoneally administered melatonin, especially prior to cerebral ischemia or during reperfusion, protects CA1 hippocampal neurons against ischemic injury. However, it has been known that severe cerebral dysfunctions, which may result in paralysis of extremities, do not occur in cases where the injury caused by cerebral ischemia is located only in hippocampus. Also, no extra-hipocampel damage was observed (Ginsburg et al., Rodent Models of Cerebral Ischemia, Stroke 20: 1627-1642 (1989)).

In addition, it has been reported that in models of cerebral ischemia induced by ligating the middle cerebral artery, the brain necroses (by observation of tissues under a microscope) of rats having no detectable level of blood melatonin after pinealectomy are significantly greater than in normal rats (Manev H. et al., FASEB J, 10(13), 1546-1551(1996)). However, this report does not suggest the role of exogenous melatonin in the presence of endogenous melatonin, since cerebral ischemia was not induced in the presence of endogenous melatonin.

It has been reported that blood (endogenous) melatonin in old men of the age of 82 to 86 is about quarter of that in young men of the age of 21 to 25 (about 80 pg/ml) (Reiter R.J., Acta Neurobiol. Exp. 54: 31-39 (1994) and Reiter R.J., FASEB J. 9: 526-533 (1995)).

On the other hand, it is described in J. Clin. Endocrinol. Metab., 61: 1214-1216 (1985); Life Sci., 37: 489-495 (1985); Br. J. Clin. Pharmacol., 19: 517-521 (1985); and Neuroendocrinology, 39: 307-313 (1984) that orally administered melatonin circulates in blood. Also, the intravenousy administered melatonin was shown to pass into the brain (Pineal Res. 5: 437-453 (1988) and Int. J. Rds. Appl. Instrum. [B] 18: 357-362 (1991)).

WO 97/20555 (corresponding to US Patent No. 5,700,828) discloses that the mild motor dysfunction of rats with a foot-fault rate of 0.01 caused by cerebral ischemia can be lowered to a foot-fault rate of 0.002 by administration of a "rescue" solution containing melatonin, kynurenine and others to the cerebral ischemic rat. The foot fault rate of the non-treated control rats was 0.005 in the experiment.

However, the transient ischemic rat model employed in WO 97/20555, wherein bilateral vertebrarterial arteriae were ligated (in practice, burned off) and one day later the bilateral internal carotid arteriae were occluded for 10 minutes (Stroke, 10: 267-272 (1979)), the rats would suffer from neuropathies (e.g., extremital peripheral nerve disturbance, a muscle disturbance, equilibrium disturbance, and visual disturbance) resulted from causes other than cerebral infarction. Ataxia would be caused by the neuropathies, leading to the lowering in foot-fault rate. In fact, no cerebral infarction could be demonstrated with other experiments in transient ischemic rats produced by the procedures as above. Further, the motor dysfunction with a foot-fault rate of 0.01 in the transient ischemic rats of WO 97/20555 is not so severe as to cause paralysis of extremities.

The foot fault rate is a known term, and is generally determined according to Hernandez-Schallert foot-fault test, wherein rats are forced to walk on a bar with a diameter of 3 to 6 cm, and the percentage of rats to fail (i.e., to slip down) is calculated. The rate is a proportion of the rats to fail after ischemia to the rats to fail before ischemia.

Accordingly, WO 97/20555 fails to suggest the treatment of cerebral infarction, especially severe cerebral infarction resulting in paralysis of extremities, or paralysis of extremities resulting from cerebral infarction.

Thus, there remains a need for an effective pharmaceutical or food composition capable of treating, paralysis of the extremities caused by cerebral infarction.

### Summary of the Invention

Accordingly, the purpose of the present invention is to provide a pharmaceutical or food composition for the treatment of paralysis of the extremities caused by cerebral infarction.

After exhaustive research, the present inventors have found that melatonin acts to treat, paralysis of the extremities caused by cerebral infarction.

Therefore, the present invention provides the use of melatonin for producing a composition for treating paralysis of extremities caused by cerebral infarction.

### Detailed Description and Preferred Embodiment

Thus, the present invention provides the use of melatonin for producing a pharmaceutical or food composition for the treatment of paralysis of the extremities caused by cerebral infarction.

The term "paralysis of extremities" as used herein means that at least one of the hands and feet is paralyzed. The term "paralysis" as used herein represents the condition where the extremities cannot be voluntarily moved, i.e., a patient suffering from the "paralysis" of hands as used herein is incapable of catching or lifting up a light article and of bringing food to the mouth, and a patient suffering from "paralysis" of the legs as used herein cannot stand even if a fixed appliance such as a handrail is used.

Therefore, the term "paralysis" as used herein is distinct from a motor disturbance where a patient suffering from paralysis of the hands can catch or lift up a light article and a patient suffering from paralysis of the legs can stand at least by using a fixed appliance, or from mild disturbances.

The term "cerebral infarction" as used herein means an infarction in any tissue or part of the brain including lacunae infarctions. Cerebral infarction is induced by cerebral ischemia or hypoxemia. As is well known, major causes of cerebral ischemia are cerebral thrombosis and cerebral embolism. Cerebral infarction itself may be an additional cause of cerebral ischemia. Cerebral infarction resulting from the condition of cerebral thrombosis may be called as "cerebral thrombosis". Also, cerebral infarction resulting from the condition of cerebral embolism may be called as "cerebral embolism". The term "cerebral infarction" as used herein includes such cerebral thrombosis and cerebral embolism as used above.

The food composition of the pharmaceutical composition can treat a patient suffering from paralysis of the extremities caused by cerebral infarction.

Melatonin may be advantageously administered, being encapsulated in an encapsulating matrix, a liposome or the like. Melatonin in an encapsulating matrix or a liposome is gradually released into the blood so that its apparent residence time in the blood is increased, leading to an increase in the bioavailability of melatonin.

Examples of suitable encapsulating matrix are cyclodextrin and pharmaceutically acceptable biodegradable synthetic or natural polymers such as polylactic acid, a copolymer of lactic acid with glycol, poly-β-hydroxybutyric acid. Cyclodextrins attached onto a pharmaceutically acceptable synthetic polymer are included in the definition of cylodextrin.

Suitable methods for preparing liposomes includes the various methods such as the vortex method (Bangham AD et al., Methods Membr. Biol. 1:-20, 1974); the ultrasonic treatment method (Johnson SM et al., Biochim. Biophys. Acta 233: 820-826, 1971); the ethanol injection method (Kremer JMH et al., Biochemistry 16: 3932-3941, 1980); the French-Press method (Hamilton et al., J. Lipid Res. 21: 981-992, 1980); the cholic acid removal method (Enoch HG et al., Proc. Natl. Acad. Sci. 76: 145-149, 1979); the ether injection method (Deamer DN, Ann, N.Y. Acad. Sci. 308: 250-258, 1987); the freeze-thaw method (Papahadjopoulos D et al., Biophim. Biophys. Acta 394: 483-491, 1975); and the reverse phase evaporation method (Szoka F et al., Proc. Natl. Acad. Sci. USA 75: 4191-4198, 1978).

The amount of melatonin to be administered to a patient having paralysis of the extremities caused by cerebral infarction depends on various factors including sex, age, body weight and diet of the patient; the administrating route; the conditions and/or degree of cerebral thrombosis and cerebral embolism; the condition and/or degree of cerebral infarction; and the like, which are taken into account by a clinician.

The daily dose of either the pharmaceutical composition or the food composition is generally determined in such a manner that the melatonin blood concentration per day ranges from 10 ng/ml to 300 µg/ml, preferably from 50 ng/ml to 100 µg/ml. The term "blood concentration per day" means the total blood concentration of melatonin administered in a day. For example, in the case when a first administration gives a blood concentration of 50 µg/ml and a second administration on the same day as the first administration gives a blood concentration of 30 µg/ml, the blood concentration per day is 80 µg/ml. The blood concentration of melatonin per day can be estimated by a clinician or a specialist based on information previously given without need of practical measurements.

Since the availability of melatonin varies depending on various factors (e.g., administration route, dosage form, encapsulation of melatonin, and age of the patient), the daily dose of the pharmaceutical composition is naturally adjusted to maintain the above blood concentration.

In the case of oral administration, melatonin should be administered to the patient in an amount of about 0.8 mg/kg to attain a melatonin blood concentration of 100 ng/ml.

When the melatonin is encapsulated in an encapsulating matrix or a liposome, its amount is expressed in terms of free (non-encapsulated) melatonin. Since melatonin in an encapsulated form is gradually released in blood, its apparent residence time in blood is longer. Thus, the blood concentration of melatonin, when melatonin is administered in an encapsulated form, may be lower than in case of the administration of melatonin in the free form.

In the case that the composition is the pharmaceutical composition, the melatonin can be administered by various routes, such as permucosally (e.g., sublingually, intranasally, oral mucosally), orally, enterally, percutaneously, intravenously, by inhalation, as a suppository, or by instillation. The administration route is determined by a clinician depending on the amount of melatonin to be administered, and the condition of the patient.

Generally, patients having paralysis of the extremities caused by cerebral infarction require a long-term treatment, and desire to be treated at home, while undergoing treatment. Oral administration of the pharmaceutical composition to such patients is preferable. In such a case, the composition of this invention is preferably given to the patient as the food composition.

The melatonin, in either case of the pharmaceutical composition or the food composition, may be administered as a single or multiple doses composition. The melatonin can be given to the patient in the form of a food composition.

When the composition is the pharmaceutical composition, the pharmaceutical composition typically comprises a pharmaceutical carrier in addition to melatonin. The "pharmaceutical carrier" as used herein includes any ingredient which is pharmaceutically acceptable and physiologically less-active.

The melatonin may be administered in the form of sustained or controlled release composition. The composition for oral administration is preferably in the form of sustained release formulation. As a sustained release composition, conventional sustained or controlled release formulations (e.g., a formulation having a gel coating and a formulation having a multiple coating) and formulations for local release (e.g., a formulation capable of rupturing in a pylorus or a formulation capable of foaming in a duodenum) are well known and may be used.

Examples of the composition for oral administration include tablets, pills, capsules, ampuls, folded powders, elixirs, suspensions, syrups and the like.

When the pharmaceutical composition is orally administered, binders such as tragacanth gum, acacia, corn starch and gelatin; vehicles such as potassium diphosphate; disintegrators such as potato starch and alginic acid; lubricants such as magnesium stearate; sweetening agents such as sucrose; dyes; flavors such as orange flavor; solvents such as water, ethanol, and glycerol can be suitably used as the pharmaceutical carrier.

When the pharmaceutical composition for oral administration comprises a pharmaceutically acceptable antioxidant such as cysteine, glutathione, ascorbic acid, sodium metasulfite, sodium bisulfite, favorable results may be obtained.

The pharmaceutical composition for oral administration may also contain nutrients such as amino acids, vitamins, lipids and glucose.

The pharmaceutical composition for injection may contain, for example, a sterile water, an isotonic saline, or a pH butter as a pharmaceutical carrier, or may be a sterile powder composition, a freeze-dried powder composition or the like, which can be used only by dissolving in a sterile water or other suitable solvent.

The pharmaceutical composition for injection may contain saccharides such as glucose, mannitol, and dextran; polyhydric alcohols such as glycerol; inorganic salts such as sodium salt and magnesium salt. Further, it may contain a pharmaceutically acceptable antioxidant such as cysteine, glutathione, ascorbic acid, sodium metasulfite, and sodium bisulfite.

When the pharmaceutical composition is administered by instillation, it may contain nutrients such as glucose, vitamins, amino acids and lipids.

Pharmaceutical carriers used in other dosage forms for intranasal, inhalation or percutaneous administrations are well known in the art.

The dosage forms and the pharmaceutical carriers mentioned above are described in Remington's Pharmaceutical Science, ed. 16 (1980), Mack Publishing Company.

Further, the pharmaceutical composition may contain any therapeutic agents for the treatment of the diseases or conditions which may induce cerebral ischemia such as cerebral thrombosis and cerebral embolism, together with melatonin.

Known therapeutic agents for cerebral embolism include an anti-edema drugs, anticoagulants, thrombolytic drugs and calcium antagonists. Known therapeutic agents for cerebral thrombosis include anti-edema drugs, anti-platelets and calcium antagonists.

Also, the pharmaceutical composition may contain a therapeutic agent used for reducing adverse influences of cerebral infarction such as a central muscle relaxant, an agent for accelerating microcirculations, and an agent for improving central metabolism.

In some cases, the pharmaceutical composition may contain an auxiliary amount of a neuroprotective, which may cooperatively function with melatonin in treatment of cerebral infarction.

The above therapeutic agents or neuroprotectives may be added to a pharmaceutical composition for oral administration of the present invention if they can be orally administered. Alternatively, they may be added to a pharmaceutical composition for parenteral administration including injection if they cannot be orally administered. An amount of each therapeutic agent or neuroprotective in the pharmaceutical composition for oral or parenteral administration can be also determined by a clinician.

In either case of the pharmaceutical composition or food composition, it is preferred to commence treatment with melatonin as quickly as possible and as soon after the cerebral infarction as possible. However, it is possible to commence treatment after the diagnosis of cerebral infarction or paralysis of the extremities, and administration of melatonin is effective after a prolonged delay of one, two, tree, or more days after the ischemic event. The melatonin therapy is typically continued until the desired level of improvement is observed in the patient.

In case of a food composition, since melatonin is tasteless and stable against heat and enzymes under cooking conditions, any food can be used. The food includes, for example, heated food such as a hamburger and a soup, unheated food such as a fruit juice and mayonnaise, fermented food such as yogurt, and cold food such as icecream and frozen food.

Liquid food (for example, gelled food such as a custard pudding and a jelly, soup, stew, and mush or gruel) is preferred for easy intake by the patient, while solid may be used depending on the conditions of the patient.

Foodstuffs, for example, seasonings such as tomato sauce, bouillon and soy sauce, are also included in the food of the invention.

When the composition is the food composition, melatonin may be contained in a food additive composition used to add melatonin to the food. The food additive composition includes, for example, a tablet or granule containing melatonin and disintegrator; a mixture comprising melatonin and an extender (such as a protein hydrolysate, starch, casein and glucose), a solution containing melatonin in, for example, an edible oil, ethanol and water), and a W/O or O/W emulsion comprising melatonin. Such a food composition may be in the form of a powder, extrudate and the like.

The addition of melatonin into food may be conducted at any suitable time. For example, melatonin may be added to a raw foodstuff before cooking such as a minced meat or cooked food such as bouillabasse and stew. Alternatively, melatonin may be added in a food before cold storing or freezing.

The amount of melatonin to be added in a food is such that it satisfies the above mentioned daily dose of melatonin.

Other features of the invention will become apparent from the following descriptions of exemplary embodiments which are given for illustration of the invention and are intended to be limiting thereof.

### Examples

### Example 1

Eight to ten-week-old male Wister rats weighing of 250 to 300 g were used.

Under halothane anesthesia, a 24G plug was inserted from a right external carotid artery of each rat so as to reach a starting site of its middle cerebral artery of the Willis' circle through its internal carotid artery. Immediately, the anesthesia was then stopped, and the rats were dehypnotized. It was confirmed that the rats rotated clockwise, had ischemia, and had paralysis of their left upper limbs. One hour later, the plug was removed from the rat under halothane anesthesia, and blood flow was thereby reperfused.

In the melatonin administration group, 1 ml of a physiological saline solution containing 6 mg/kg of melatonin was injected using a probe into a stomach of each rat each day for 10 days starting 24 hours after the reperfusion. In the control group, 1 ml of a physiological saline solution was similarly injected into each rat.

After 19 days, the presence of adduction/pronation of left upper limb and the clockwise rotation were determined for each rat.

To assess the clockwise rotation, each rat was placed on a round plate 2m in diameter for 5 minutes. If the rat had paralysis at left upper limb, it rotated clockwise.

To assess the adduction/pronation of the left upper limb, each rat was hung via its tail, and the movement of the left upper limb was observed. If the rat could not voluntarily move the left upper limb, and had paralysis of the left upper limb, the adduction/pronation of the left upper limb was observed.

The results are shown in Table 1.

**Table 1**

| | during ischemia | 19 days after ischemia |
|---|---|---|
| Adduction/pronation | | |
| Control group | 4/4 | 3/4 |
| Melatonin administration group | 4/4 | 1/4 |

| Clockwise rotation | | |
|---|---|---|
| Control group | 4/4 | 3/4 |
| Melatonin administration group | 4/4 | 1/4 |

It is clear from the results in Table 1 that the melatonin administration group showed significantly less adduction/pronation of left upper hand and less clockwise rotation as compared with the control group in 19 days after ischemia.

### Example 2

In the experiment similar to that as described in Example 1, the adduction/pronation of left upper limb and the clockwise rotation were determined in 11 days after the ischemic incidence.

The results are shown in Table 2.

**Table 2**

| | during ischemia | 11 days after ischemia |
|---|---|---|
| adduction/pronation | | |
| control group | 7/7 | 5/7 |
| melatonin administration group | 7/7 | 3/7 |

| clockwise rotation | | |
|---|---|---|
| control group | 7/7 | 2/7 |
| melatonin administration group | 7/7 | 0/7 |

### Example 3

Using the rats from the experiment of Example 2, the area of the cerebral infarcted lesion in each rat in 11 days was determined.

The area of the cerebral infarcted lesion was determined by preparing a serial section of 50 µm from a cerebral infarcted region of about 3 mm and subjecting the section showing a maximum lesion to the NIH measurement program to determine the area of the maximum lesion.

The results with respect to the area of the infarcted lesion are shown in Table 3.

**Table 3**

| | striatum | cerebral cortex |
|---|---|---|
| control group | 10.62 mm² | 14.53 mm² |
| melatonin administration group | 7.15 mm² | 5.47 mm² |

It is clear from the results in Table 3 that the melatonin administration group showed less brain damage in both the striatum and the cerebral cortex as compared with the control group. The area of the cerebral infarcted lesion, especially in cerebral cortex was smaller.

### Effect of the Invention

As seen in Tables 1 and 2, the number of rats cured of paralysis of the left upper limb (caused by cerebral infarction through cerebral ischemia) by the treatment with the pharmaceutical composition of the present invention was significantly greater than the number of rats spontaneously cured without the treatment. In WO97/20555, the percentage of rats spontaneously cured of the mild motor dysfunction with a foot fault rate of 0.01 was 50%. This percentage is greater than the percentage of rats (30%) cured by the treatment with the "rescue" solution of WO97/20555.

Thus, mild motor disturbances as disclosed in WO97/20555 could be rather effectively treated by phsyicotherapies including a motorpathy. It is believed that melatonin gives a minor contribution to the treatment of mild motor disturbances. On the other hand, administration of melatonin is very effective in the treatment of paralysis of extremities caused by cerebral infarction, as compared with any other available method. Thus, melatonin gives a major contribution to the treatment of paralysis of extremities caused by cerebral infarction.

Accordingly, the pharmaceutical composition for the treatment of paralysis of extremities has very high advantages as compared with known pharmaceutical compositions for the treatment of mild motor disturbances caused by cerebral ischemia.

## Claims

1. Use of melatonin for producing a composition for the treatment of paralysis of the extremities caused by cerebral infarction.

2. The use of claim 1, wherein the composition is a pharmaceutical composition.

3. The use of claim 2, wherein the pharmaceutical composition is for oral administration.

4. The use of claim 2, wherein melatonin is encapsulated in an encapsulating matrix or a liposome.

5. The use of claim 1, wherein the composition is a food composition.

6. The use of claim 1, wherein the composition is for administration to a subject in the form of a food such that melatonin is contained in the food or in a food additive composition added to the food, said food additive composition comprising melatonin together with an additive.

## Patentansprüche

1. Verwendung von Melatonin zur Herstellung einer Zusammensetzung für die Behandlung von durch einen zerebralen Infarkt verursachten Lähmungen der Extremitäten.

2. Verwendung nach Anspruch 1, wobei die Zusammensetzung eine Arzneimittelzusammensetzung ist.

3. Verwendung nach Anspruch 2, wobei die Arzneimittelzusammensetzung für die orale Verabreichung vorgesehen ist.

4. Verwendung nach Anspruch 2, wobei Melatonin in eine Verkapselungsmatrix oder einem Liposom eingekapselt ist.

5. Verwendung nach Anspruch 1, wobei die Zusammensetzung eine Nahrungsmittelzusammensetzung ist.

6. Verwendung nach Anspruch 1, wobei die Zusammensetzung für die Verabreichung an einen Patienten in der Form eines Nahrungsmittels vorgesehen ist, so dass Melatonin in dem Nahrungsmittel oder in einer zu dem Nahrungsmittel gegebenen Nahrungsmittelzusatzzusammensetzung enthalten ist, wobei die Nahrungsmittelzusatzzusammensetzung Melatonin zusammen mit einem Zusatz enthält.

## Revendications

1. Utilisation de mélatonine pour la production d'une composition destinée au traitement de la paralysie des extrémités provoquée par un infarctus cérébral.

2. Utilisation suivant la revendication 1, dans laquelle la composition est une composition pharmaceutique.

3. Utilisation suivant la revendication 2, dans laquelle la composition pharmaceutique est destinée à l'administration orale.

4. Utilisation suivant la revendication 2, dans laquelle la mélatonine est encapsulée dans une matrice d'encapsulation ou un liposome.

5. Utilisation suivant la revendication 1, dans laquelle la composition est une composition alimentaire.

6. Utilisation suivant la revendication 1, dans laquelle la composition est destinée à l'administration à un sujet sous forme d'un aliment de telle sorte que la mélatonine soit présente dans l'aliment ou dans une composition d'additif alimentaire ajoutée à l'aliment, ladite composition d'additif alimentaire comprenant de la mélatonine conjointement avec un additif.
